# EUROPEAN PATENT APPLICATION

(11) **EP 4 268 894 A1**
(43) Date of publication of application: **01.11.2023**
(21) Application number: 21915296.4
(22) Date of filing: 27.12.2021
(51) Int. Cl.: A61P 21/00, A61P 43/00, A61K 31/7068, A61K 31/7072, A23L 33/10

(54) **PGC-1alpha EXPRESSION PROMOTING AGENT, MUSCLE-BUILDING AGENT, AND MITOCHONDRIA ACTIVATING AGENT**

(30) Priority: 28.12.2020 JP 2020218128
(71) Applicant: YAMASA CORPORATION, Choshi-shi, Chiba 288-0056 (JP)
(72) Inventor: NAKAGAWARA,Kosuke, Choshi-shi, Chiba 288-0056 (JP); TAKEUCHI,Chieri, Choshi-shi, Chiba 288-0056 (JP); ISHIGE,Kazuya, Choshi-shi, Chiba 288-0056 (JP)
(74) Representative: Maiwald GmbH
(86) International application number: PCT/JP2021/048701
(87) International publication number: WO 2022/145438

(57) **Abstract**

The present invention relates to a PGC-1α expression promoting agent, a muscle building agent, or a mitochondria activating agent, which comprises at least one pyrimidine nucleotide or a precursor thereof as an active ingredient.

## Description

### [Technical Field]

The present invention relates to a PGC-1α expression promoting agent, a muscle-building agent and a mitochondria activating agent.

### [Background Art]

Muscle is formed by myocyte differentiation. In the myocyte differentiation, myoblasts derived from satellite cells fuse with each other to form multinuclear fused bodies called myotube cells. The myotube cells are then assembled and aligned to form myofibers, which will be tissues generating large force as muscle.

It is known that a group of transcription factors specific to each stage of the myogenic differentiation process is specifically expressed and function, and Pax 7 functions in satellite cells, Myf5 and MyoD function in myoblasts, and Myogenin functions during myotube cell formation. To promote myogenesis, it is important to increase factors involved in muscle differentiation.

Muscle is roughly classified into slow twitch muscular fibers (type I fibers) and fast twitch muscular fibers (type II fibers), and fast twitch muscular fibers are further classified into subtypes such as type IIa fibers, type IIb fibers, and type IIx fibers. The type I fibers have a slower contracting rate, but they have superior endurance, more mitochondria and antioxidants, and more capillaries adjacent to muscle fibers. In contrast, the type II fibers have a higher contracting rate, but they have less endurance, fewer mitochondria and antioxidants, and fewer capillaries adjacent to muscle fibers.

PGC-1a (Peroxisome proliferator-activated receptor gamma coactivator 1-alpha) is a factor that plays a central role in regulating myofiber types. The PGC-1α was found as a transcriptional coactivator that activates transcription by the intranuclear receptor PPARγ in brown adipocytes. It is known that the PGC-1α is expressed not only in brown adipocytes but also in many tissues such as skeletal muscle, heart, kidney and brain, and regulates biosynthesis of mitochondrial and energy production.

Based on these functions of the PGC-1α, mice overexpressing PGC-1α ameliorate age-related symptoms such as decreased muscle mass, motor function, bone density, mitochondrial function, exacerbated insulin resistance and systemic inflammatory response, and obesity, thereby live longer. (Non-Patent Literature 1).

On the other hand, PGC-1a knockout mice are known to develop neuropathy in addition to muscle dysfunction and obesity (Non-Patent Literature 2). It is also known that neurodegenerative diseases such as Alzheimer's disease, Parkinson's disease, and Huntington's disease are associated with abnormalities and decreased expression of the PGC-1α gene. Thus, activation of PGC-1α is emerging as a novel method for treating a wide variety of neurodegenerative diseases.

Since increased expression of PGC-1α is predicted to have effects on the above-mentioned diseases or symptoms, there is also prior art relating to the action of increasing PGC-1α expression. For example, Patent Literature 1 discloses that Helipyrone A promotes PGC-1α production in neurons and has effects of improving various neurotransmission disorders and short-term memory disorders in which the PGC-1α is involved.

Patent Literature 2 also discloses that the combination of β-hydroxy-β-methylbutyrate (HMB) and a secondary ingredient enhances the expression of myogenin, a myotube cell differentiation marker, and shows a significant muscle-building effect.

Further, Patent Literature 3 discloses that pyrroloquinoline quinone exhibits mitochondria activating effects.

Cytidylic acid and uridylic acid are a type of nucleotide and are widely included in living organisms and foods, which can be highly safe materials.

Patent Literature 4 discloses an agent for improving emotional disorders characterized by containing nucleotides. Patent Literature 4 also discloses an immunostimulant characterized by containing nucleotides. Further, Non-Patent Literature 3 discloses that rats which have administered a mixture of cytidylic acid and uridylic acid tolerated prolonged exercise on a treadmill.

However, the physical strength improvement described in the above Non-Patent Literature 3 is only by measuring the improvement of the ability to continuously perform a certain exercise condition such as treadmill, and the amount of biochemical parameters related to fatigue such as glycogen and lactic acid in liver and muscle, but it does not analyze any expression of transcription factors such as PGC-1α that correlate with increased muscle mass. Therefore, the above Non-Patent Literature 3 does not examine muscle building effects such as promotion of myotube cell differentiation at all.

Therefore, conventionally, it has not been evaluated whether nucleic acid-related substances such as cytidylic acid and uridylic acid have the ability to promote the PGC-1a expression, the ability to promote myotube cell differentiation and/or the ability to activate mitochondria.

### [Citation List]

### [Patent Literatures]

[PTL 1]
   Japanese Patent Application Publication No. 2017-043566 A
[PTL 2]
   Japanese Patent Application Publication No. 2018-090504 A
[PTL3]
   WO 2006/025247 A1
[PTL 4]
   Japanese Patent Application Publication No. H10-203989 A
[PTL 5]
   Japanese Patent Application Publication No. 2001-314172 A

### [Non-Patent Literatures]

[NON-PTL 1]
   Tina Wenz et al., "Increased muscle PGC-1a expression protects from sarcopenia and metabolic disease during aging" PNAS December 1, 2009, 106 (48) 20405-20410
[NON-PTL 2]
   Teresa C Leone, et al., "PGC-1α Deficiency Causes Multi-System Energy Metabolic Derangements: Muscle Dysfunction, Abnormal Weight Control and Hepatic Steatosis", PLoS Biol 3(4): e101.
[NON-PTL 3]
   Gella, A, et al., "Effect of the nucleotides CMP and UMP on exhaustion in exercise rats", J Physiol Biochem, 64(1), 9-18, 2008

### [Summary of Invention]

### [Problem to be Solved by the Invention]

An object of the present invention is to provide a novel and highly safe PGC-1a expression promoting agent, muscle-building agent or mitochondria activating agent that has effects of promoting PGC-1α expression and exerting muscle building when it is used as a food, feed, drug, quasi drug, and the like.

### [Methods for Solving the Problem]

As a result of intensive studies to achieve the above object, the present inventors have first found that pyrimidine nucleotides or their precursors have clear effects of promoting PGC-1α expression, promoting myotube cell differentiation, and activating mitochondria, in addition to anti-fatigue and physical strength improving effects conventionally known in the art, and have an effect of significantly promoting muscle building, and they have completed the present invention.

In other words, the present invention is a PGC-1α expression promoting agent, a muscle building agent, or a mitochondria activating agent, which comprises at least one pyrimidine nucleotide or a precursor thereof as an active ingredient.

### [Effects of Invention]

The PGC-1α expression promoting agent according to the present invention promotes differentiation of myotube cells and achieves muscle building. The achievement of muscle building provides a new means for improving exercise performance and improving the quality of life (QOL) of patients and elderly persons suffering from muscle weakness. Further, it is known that age-related muscle atrophy is suppressed in mice overexpressing PGC-1a (Non-Patent Literature 1), and an effect of suppressing the muscle weakness is also expectable by promoting a PGC-1α expression.

Diseases or symptoms caused by low expression of PGC-1α include diabetes, dyslipidemia, obesity, impaired brain function, neurodegenerative diseases, aging, and the like. The PGC-1α expression promoting agent according to the present invention may provide a new means for preventing and treating the diseases or symptoms caused by low expression of PGC-1α.

### [Brief Description of Drawings]

[Fig. 1]

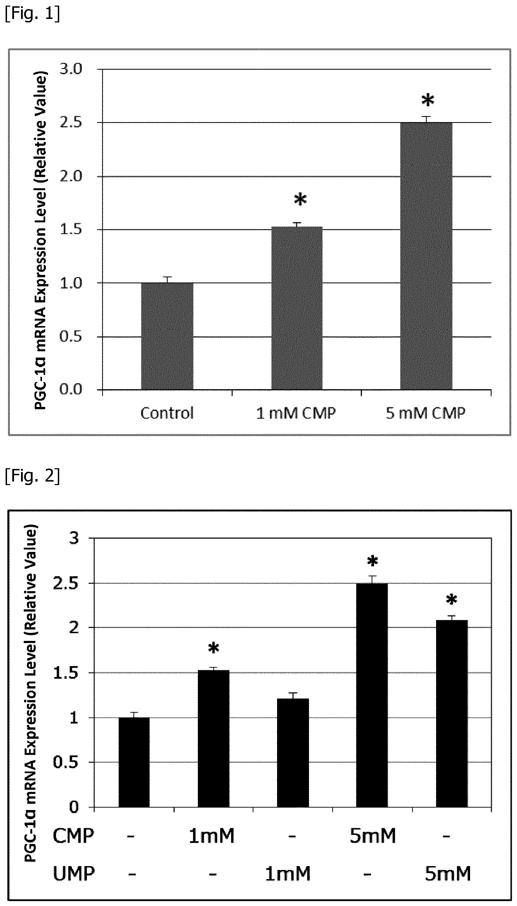

   Fig. 1 shows an effect of cytidylic acid on the promotion of PGC-1α expression in C2C12 cells according to Example 1. In the figure, the CMP means disodium cytidylate, the error bar means standard error, ^{∗} means p < 0.05.
[Fig. 2]
   Fig. 2 shows effects of cytidylic acid and uridylic acid on the promotion of PGC-1α expression in C2C12 cells according to Example 2. In the figure, the CMP means disodium cytidylate, the UMP means disodium uridylate, the error bar means standard error, and the * means p < 0.05.
[Fig. 3]

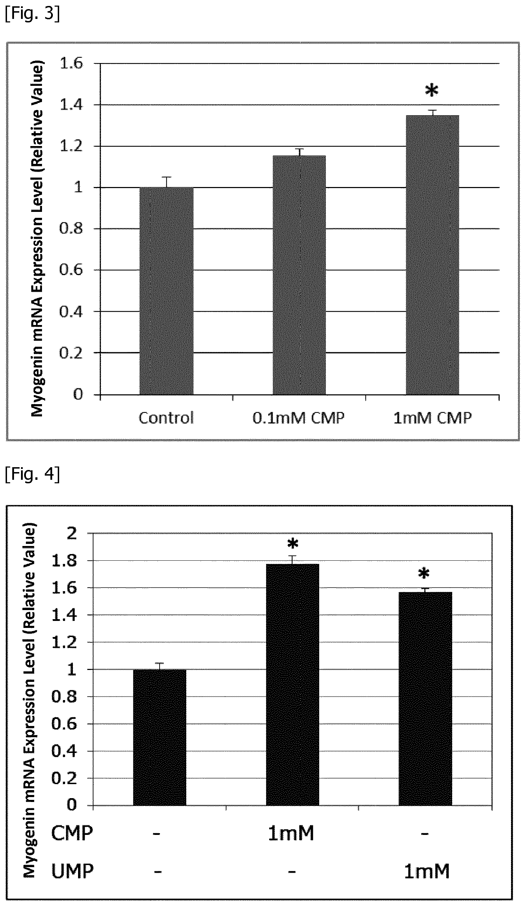

   Fig. 3 shows an effect of cytidylic acid on the promotion of Myogenin expression in C2C12 cells according to Example 3. In the figure, the CMP means disodium cytidylate, the error bar means standard error, and the * means p < 0.05.
[Fig. 4]
   Fig. 4 shows effects of cytidylic acid and uridylic acid on the promotion of Myogenin expression in C2C12 cells according to Example 4. In the figure, the CMP means disodium cytidylate, the UMP means disodium uridylate, the error bar means standard error, and the * means p < 0.05.
[Fig. 5]

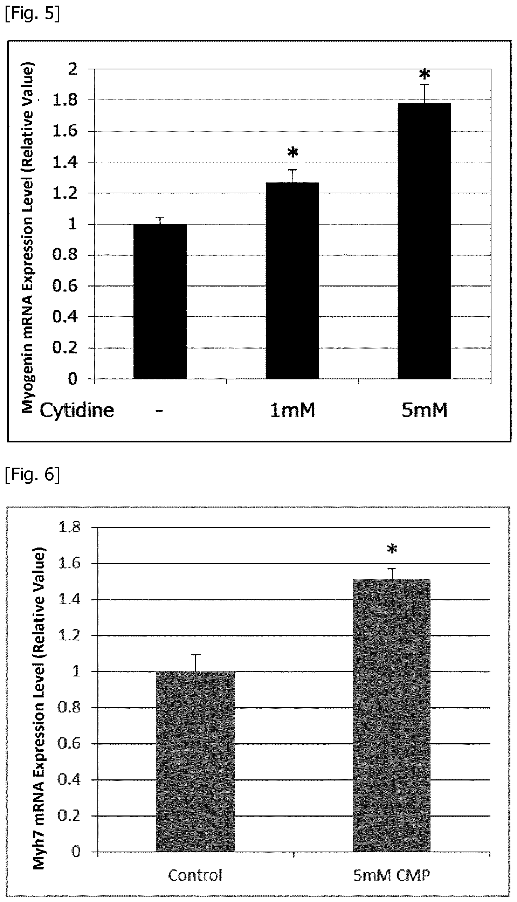

   Fig. 5 shows an effect of cytidine on the promotion of Myogenin expression in C2C12 cells according to Example 4. In the figure, the error bar means standard error, and the * means p < 0.05.
[Fig. 6]
   Fig. 6 shows an effect of cytidylic acid on the promotion of Myh7 (slow-muscle type myosin heavy chain) expression in C2C12 cells according to Example 5. In the figure, the CMP means disodium cytidylate, the error bar means standard error, and the * means p < 0.05.
[Fig. 7]

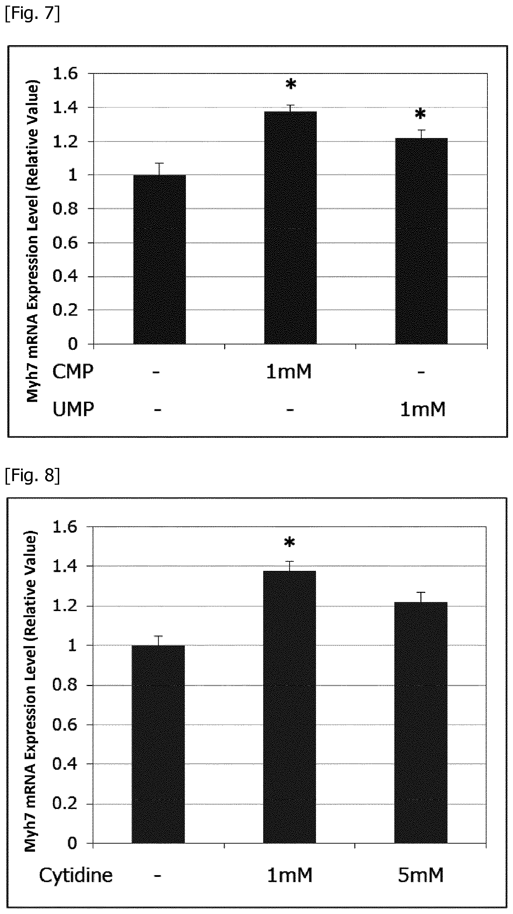

   Fig. 7 shows effects of cytidylic acid and uridylic acid on the promotion of Myh7 (slow-muscle type myosin heavy chain) expression in C2C12 cells according to Example 6. In the figure, the CMP means disodium cytidylate, the UMP means disodium uridylate, the error bar means standard error, and the * means p < 0.05.
[Fig. 8]
   Fig. 8 shows an effect of cytidine on the promotion of Myh7 (slow-muscle type myosin heavy chain) expression in C2C12 cells according to Example 6. In the figure, the error bar means standard error, and the * means p < 0.05.
[Fig. 9]

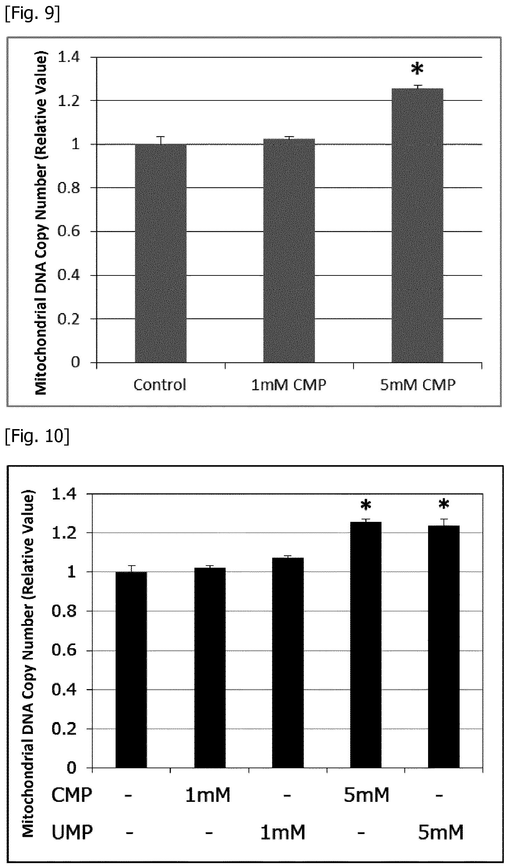

   Fig. 9 shows an effect of cytidylic acid on an increase in mitochondrial DNA copy number in C2C12 cells according to Example 7. In the figure, the CMP means disodium cytidylate, the error bar means standard error, and the * means p < 0.05.
[Fig. 10]
   Fig. 10 shows effects of cytidylic acid and uridylic acid on an increase in mitochondrial DNA number in C2C12 cells according to Example 8. In the figure, the CMP means disodium cytidylate, the UMP means disodium uridylate, the error bar means standard error, and the * means p < 0.05.
[Fig. 11]

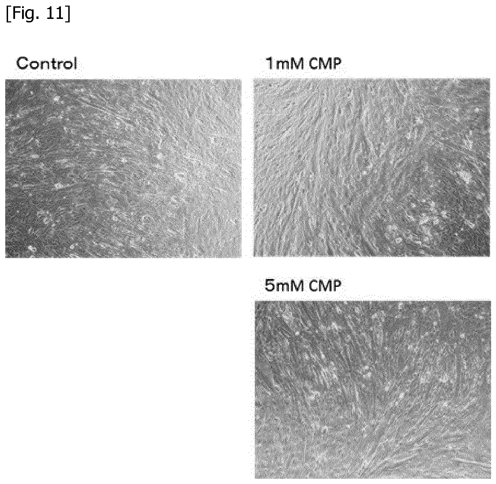

   Fig. 11 shows an effect of cytidylic acid on an increase in myotube cell diameter in C2C12 cells according to Example 9 at magnifications of 100. In the figure, the CMP means disodium cytidylate.
[Fig. 12]

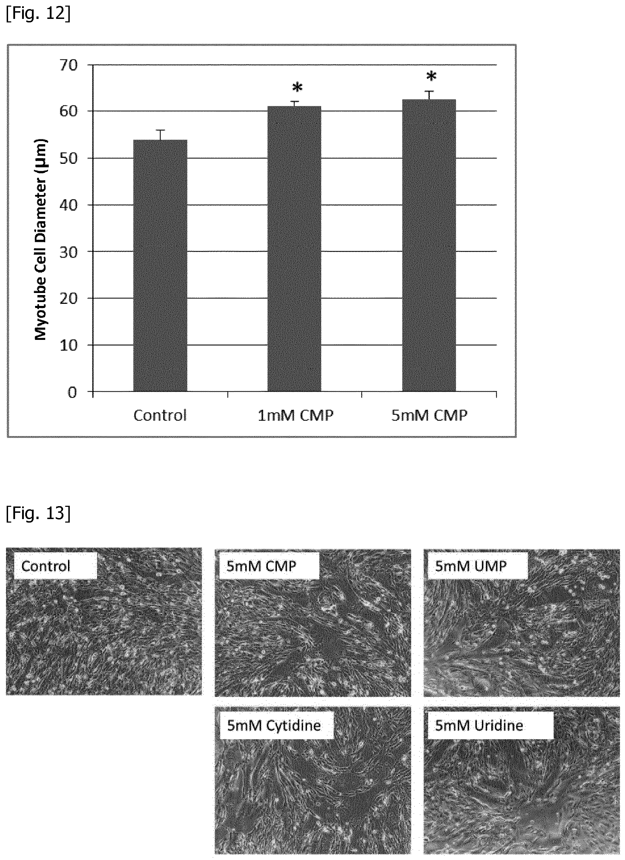

   Fig. 12 shows an effect of cytidylic acid on an increase in myotube cell diameter in C2C12 cells according to Example 9. In the figure, the CMP means disodium cytidylate, the error bar means standard error, and the * means p < 0.05.
[Fig. 13]
   Fig. 13 shows effects of cytidylic acid, uridylic acid, cytidine, and uridine on an increase in myotube cell diameter in C2C12 cells according to Example 10. In the figure, the CMP means disodium cytidylate and the UMP means disodium uridylate.
[Fig. 14]

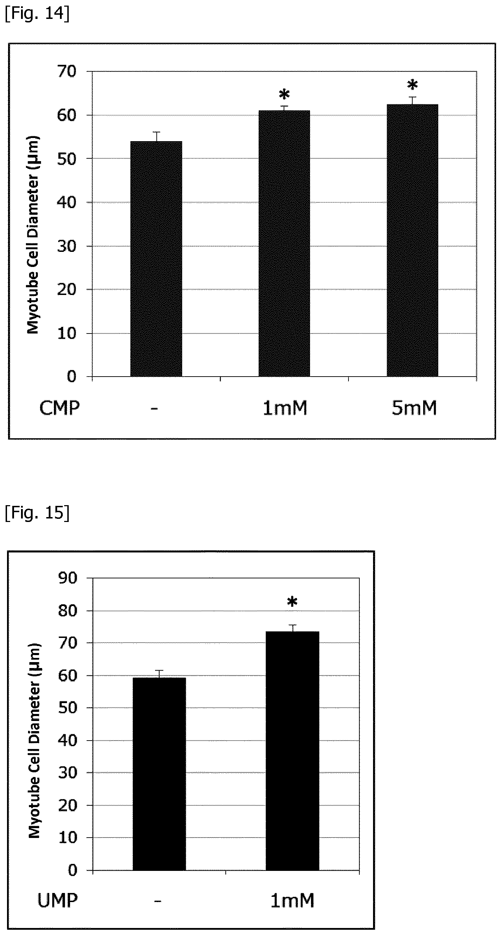

   Fig. 14 shows an effect of cytidylic acid on an increase in myotube cell diameter in C2C12 cells according to Example 10. In the figure, the CMP means disodium cytidylate, the error bar means standard error, and the * means p < 0.05.
[Fig. 15]
   Fig. 15 shows an effect of uridylic acid on an increase in myotube cell diameter of C2C12 cells according to Example 10. In the figure, the UMP means disodium uridylate, the error bars means standard error, and the * means p < 0.05.
[Fig. 16]

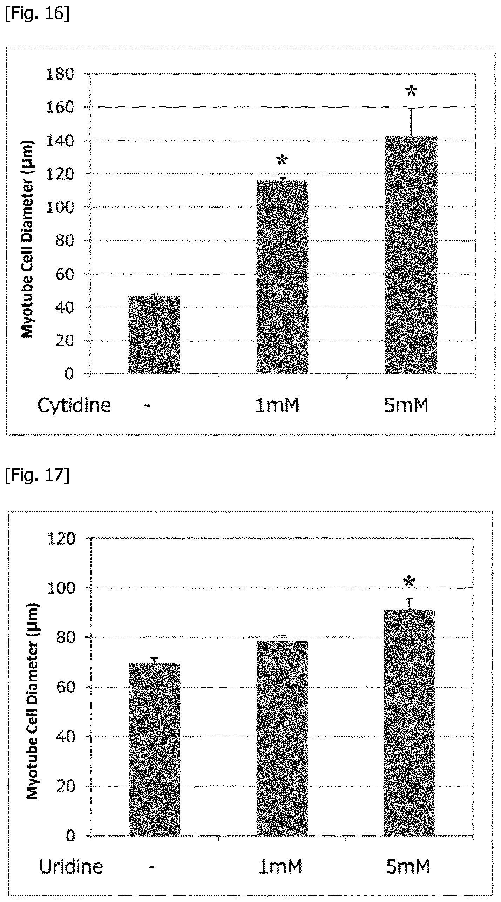

   Fig. 16 shows an effect of cytidine on an increase in myotube cell diameter in C2C12 cells according to Example 10. In the figure, the error bar means standard error, and the * means p < 0.05.
[Fig. 17]
   Fig. 17 shows an effect of uridine on an increase in myotube cell diameter in C2C12 cells according to Example 10. In the figure, the error bar means standard error, and the * means p < 0.05.

### [Detailed Description of the Invention]

The present invention relates to a PGC-1α expression promoting agent, a muscle-building agent, or a mitochondria activating agent, which contains a pyrimidine nucleotide or a precursor thereof as an active ingredient. Hereinafter, when simply referring to an "agent", it is a generic term for the above PGC-1α expression promoting agent, muscle-building agent, and mitochondria activating agent. In the agent according to the present invention, the PGC-1a expression promoting action, the muscle-building action, and the mitochondria activating action may occur simultaneously in correlation with one another.

As used herein, the PGC-1α expression promoting means a statistically significant increase in a measured amount of mRNA as compared to a control, when the PGC-1α expression-promoting agent according to the present invention is administered.

Since PGC-1α is known to be associated with various diseases or symptoms such as muscle atrophy, diabetes, dyslipidemia, obesity, impaired brain function, neurodegenerative diseases, and aging, the PGC-1α expression promoting agent according to the present invention may contribute to alleviating, suppressing, or preventing these diseases or symptoms.

As used herein, the "muscle-building" means that at least one of the following effects (1) to (3) is provided when the muscle-building agent according to the present invention is administered, so that muscle fibers become thicker to strengthen the muscle:
(1) expression of genes related to myotube cell differentiation, represented by Myogenin or the like, is promoted, so that myotube cell differentiation is promoted;
(2) expression of genes related to muscle-forming proteins, represented by Myh7 or the like, is promoted; and
(3) diameters of myotube cells are significantly increased.

The mitochondria activating as used herein refers to events such as an increase in mitochondrial DNA copy number, which can be evaluated by using known methods such as real-time PCR as an indicator of the degree of activation.

As used herein, the pyrimidine nucleotide means cytidylic acid and uridylic acid.

The cytidylic acid (cytidine monophosphate, cytidine 5'-phosphate, CMP) is a compound represented by CAS Registry Number 63-37-6. When cytidylic acid is mentioned herein, salts of cytidylic acid are also included.

When a mass of cytidylic acid is described herein, it represents a mass when converted to disodium cytidylate (CMP,2Na). When a concentration (%) of cytidylic acid is mentioned herein, it is a mass volume percent concentration (w/v%) unless otherwise specified, and a mass converted to CMP,2Na is used as the mass of cytidylic acid. If a salt other than the disodium salt is selected, or if it is a free acid that does not form a salt, it is a mass when converted to CMP,2Na, based on the substance material of the cytidylic acid.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to disodium uridylate (UMP,2Na). When a concentration (%) of uridylic acid is mentioned herein, it is a mass volume percent concentration (w/v%) unless otherwise specified, and a mass converted to UMP,2Na is used as the mass of uridylic acid. If a salt other than the disodium salt is selected, or if it is a free acid that does not form a salt, it is a mass when converted to UMP,2Na, based on the substance material of the uridylic acid.

As used herein, a pyrimidine nucleotide precursor means a compound that can be metabolized to the pyrimidine nucleotide, i.e., cytidylic acid and/or uridylic acid. Whether a compound is included in the pyrimidine nucleotide precursor is determined by the presence or absence of knowledge that the compound is converted to the pyrimidine nucleotide. Specifically, cytidine diphosphate, cytidine triphosphate, uridine diphosphate, and uridine triphosphate, which are known to be degraded to cytidylic acid and/or uridylic acid by the action of ectonucleotidases and like (Isao Matsuoka, "Ectonucleotidases in Nervous System", Clinical Chemistry 33: 11-18, 2004), and cytidine, cytosine, uridine, and uracil, which are known to be phosphorylated to cytidylic acid and/or uridylic acid by the action of kinases (A Orengo, "Regulation of enzymic activity by metabolites. I. Uridine-cytidine kinase of Novikoff ascites rat tumor", J Biol Chem. 1969 Apr 25; 244(8): 2204-9.) are exemplified as pyrimidine nucleotide precursors as used herein.

Examples of the pyrimidine nucleotides or precursors thereof in the present invention include, as described above, cytidine, cytosine, cytidylic acid, cytidine diphosphate, cytidine triphosphate, uridine, uracil, uridylic acid, uridyl diphosphate, and uridyl triphosphate. Among them, cytidylic acid, uridylic acid, cytidine, and uridine are preferred.

The concept of cytidylic acid as used herein includes salts as described above. The salts of cytidylic acid include alkali metal salts such as sodium and potassium salts; alkaline earth metal salts such as calcium, magnesium and barium salts; basic amino acid salts such as arginine and lysine; ammonium salts such as ammonium and tricyclohexylammonium salts; and various alkanolamine salts such as monoethanolamine salts, diethanolamine salts, triethanolamine salts, monoisopropanolamine salts, diisopropanolamine salts and triisopropanolamine salts. Preferably, the salts may be alkali metal salts such as sodium salts. Specific examples of such alkali metal salts include monosodium cytidylate and disodium cytidylate, disodium cytidylate being preferred from the standpoint of handleability.

The concept of uridylic acid as used herein includes salts as described above. The salts of uridylic acid include alkali metal salts such as sodium and potassium salts; alkaline earth metal salts such as calcium, magnesium and barium salts; basic amino acid salts such as arginine and lysine; ammonium salts such as ammonium and tricyclohexylammonium salts; and various alkanolamine salts such as monoethanolamine salts, diethanolamine salts, triethanolamine salts, monoisopropanolamine salts, diisopropanolamine salts and triisopropanolamine salts. Preferably, the salts may be alkali metal salts such as sodium salts. Specific examples of such alkali metal salts include monosodium uridylate and disodium uridylate, disodium uridylate being preferred from the standpoint of handleability.

The above active ingredients may be used alone or in combination of two or more.

There is no particular limitation on the origins of the active ingredients, and those derived from natural products such as yeast, bacteria, seafood, animals, and plants are suitable.

The agents according to the present invention can be used for practical purposes as compositions for food and drink products, supplements, prepared milk powder, enteral nutritional supplements, healthy food and drink products (including Food for specified health uses and Food with function claims), additives for animal feed, and pharmaceutical products for humans or animals other than humans.

When the agent according to the present invention is provided as the food and drink product, health food and drink product or prepared milk powder, it can be made into a food or drink product having a PGC-1α expression-promoting function, a muscle-building function or a mitochondria activating function by adding the above active ingredients to the known food and drink product as appropriate. The food and drink products of interest include milk and dairy products, seasonings, beverages, confectioneries, breads, noodles, oils and fats, processed meat products, processed marine products, processed agricultural products, frozen foods, and instant foods.

The active ingredients as described above can also be mixed with materials for the food and drink products to produce novel food and drink products that have the PGC-1α expression promoting effect, muscle building effect, or mitochondria activating effect. The shape of the food and drink products of interest can be selected from various forms, such as tablets, granules, capsules, powders, solutions, syrups, emulsions, and pastes. In addition to the active ingredients according to the present invention, various excipients and seasoning ingredients that can be used as foods may be added as needed in the production of those food products.

The food and drink product as described above may be provided and sold as a food and drink product labeled with the health application which is the PGC-1α expression promoting effect, muscle-building effect, or mitochondria activating effect. The act of "labeling" includes all acts to make the above application known to users, and all expressions that may evoke or analogize the above application fall under the act of "labeling" in this invention, regardless of the purpose of the labeling, the content of the labeling, and the object or medium to be labeled.

It is preferable that the above "labeling" be made by means of expressions that enable users to directly recognize the above application. Specific examples include the act of assigning, delivering, displaying for the purpose of assignment or delivery, or importing of goods or packages of the goods in relation to the food and drink products, which describe the above application, or the act of displaying or distributing advertisement materials, price lists or transaction documents in relation to the goods, which describes the above application, and the act of providing information about these contents, which describes the above application, through electromagnetic means (e.g., through the internet).

It is preferable that the contents of the labeling are those approved by the government or the like (e.g., labeling that has been approved based on various systems established by the government and is performed in a manner based on such approval). It is also desirable to attach such labeling to packages, containers, catalogs, pamphlets, POP, and other promotional materials at the places of sales, and other documents.

When the agents according to the present invention are practically provided as pharmaceuticals, supplements, enteral nutritional products, and the like, the above active ingredient can be formulated alone or in combination with formulation aids or the like. The formulation may be orally or parenterally administered, and it is preferably orally or enterally administrated.

The formulations as described above can be tablets, granules, capsules, granules, dispersions, solutions, syrups, emulsions, and the like for oral administration, and injections, sprays, ointments, patches, and the like, for parenteral administration.

In addition to the active ingredients according to the present invention, other formulation aids such as excipients, binders, disintegrants, lubricants, taste masking agents, dissolution aids, suspending agents, coating agents, and the like, may be used in combination as appropriate according to each dosage form.

The amount of the above active ingredient in the formulation according to the present invention may be appropriately selected from the range of 0.1 to 30% (w/w), depending on the purpose of use (prevention, health or symptom relief, etc.), age of the subject, method of administration or intake, dosage form, and the like.

The amount of administration or intake of the agent according to the present invention may be appropriately selected from the range of 1 mg to 800 g per a day, although it will vary depending on the subject's age, weight, degree of symptoms, the method of administration or intake, and the like.

### [Examples]

The present invention will be more specifically described in the following Examples, but these Examples are not to be construed as limiting the technical scope of the present invention.

### (Example 1) PGC-1a Gene Expression Promoting Effect (I)

Mouse myoblast cell line C2C12 cells (RIKEN BRC, RCB0987) were suspended in growth medium (Dulbecco's modified Eagle's Medium supplemented with 10% FBS, 100 units/ml of penicillin, 100 µg/ml of streptomycin), seeded in 24-well plates, and incubated in a 37°C, 5% COz incubator until the cell density reached 70-90%. The growth medium was removed, and exchanged to a differentiation induction medium (Dulbecco's modified Eagle's Medium supplemented with 2% adult bovine serum, 100 units/ml of penicillin, 100 µg/ml of streptomycin) containing 1 mM disodium cytidylate or 5 mM disodium cytidylate, or to a differentiation induction medium that did not contain disodium cytidylate. After culturing the cells for 6 days while exchanging the medium once every 2 days, the medium was removed, and total RNA was extracted from the cells using the RNeasy Mini kit (QIAGEN). Unless otherwise stated, each sample n=6 was used for the study, including the following Examples.

Using the total RNA as a template, a reverse transcription reaction solution was prepared using the ReverTra Ace(R) qPCR RT Kit (Toyobo). The reverse transcription reaction solution, GoTaq(R) qPCR Master Mix (Promega), and a real-time PCR device, Thermal Cycler Dice Real Time System (Takara Bio), were used to measure mRNA expression levels of PGC-1a and β-actin as an internal standard. Analysis was performed by relative quantification, and mRNA expression levels were corrected using the β-actin mRNA expression level as an endogenous control. Dunnett's multiple comparison test was performed for statistical analysis. The threshold of statistical significance was set to 5%. The results are shown in Fig. 1.

As shown in Fig. 1, the PGC-1α mRNA expression level was concentration-dependently and remarkably enhanced by cytidylic acid. These results indicate that cytidylic acid has an excellent PGC-1α expression promoting effect.

### (Example 2) PGC-1α Gene Expression Promoting Effect (II)

Mouse myoblast cell line C2C12 cells (RIKEN BRC, RCB0987) were suspended in growth medium (Dulbecco's modified Eagle's Medium supplemented with 10% FBS, 100 units/ml of penicillin, 100 µg/ml of streptomycin), seeded in 24-well plates, and incubated in a 37°C, 5% COz incubator until the cell density reached 70-90%. The growth medium was removed, and exchanged to a differentiation induction medium (Dulbecco's modified Eagle's Medium supplemented with 2% adult bovine serum or 2% horse serum, 100 units/ml of penicillin and 100 µg/ml of streptomycin as antibiotics) containing test substances, or to a differentiation induction medium that did not contain the test substances. After culturing the cells for 4-6 days while exchanging the medium once every 2 days, the medium was removed, and total RNA was extracted from the cells using the RNeasy Mini kit (QIAGEN).

Using the total RNA as a template, a reverse transcription reaction solution was prepared using the ReverTra Ace(R) qPCR RT Kit (Toyobo). The reverse transcription reaction solution, GoTaq(R) qPCR Master Mix (Promega), and a real-time PCR device, Thermal Cycler Dice Real Time System (Takara Bio), were used to measure the mRNA expression levels of PGC-1α and β-actin as an internal standard. Analysis was performed by relative quantification, and the mRNA expression level was corrected using the β-actin mRNA expression level as an endogenous control. Statistical analysis was performed by t-test or Dunnett's multiple comparison test using the differentiation induction medium treatment group without the test substances as a control. The threshold of statistical significance was set to 5%. The results are shown in Fig. 2.

As shown in Fig. 2, the PGC-1a mRNA expression level was concentration-dependently and remarkably enhanced by the treatment with cytidylic acid and the uridylic acid. These results indicate that cytidylic acid and uridylic acid have an excellent PGC-1α expression promoting effect.

### (Example 3) Myogenin Gene Expression Promoting Effect (I)

A level of Myogenin mRNA expression, a marker gene for myotube cell differentiation, was evaluated by the following method:
Mouse myoblast cell line C2C12 cells were suspended in growth medium, seeded in 24-well plates, and incubated in a 37°C, 5% CO₂ incubator until the cell density reached 70-90%. The growth medium was removed, and exchanged to a differentiation induction medium containing 1 mM disodium cytidylate or 1 mM disodium cytidylate or containing no cytidylic acid. The cells were cultured for 6 days while exchanging the medium once every 2 days. Total RNA extraction and reverse transcription reaction were performed as in Example 1. Real-time PCR analysis was used to determine mRNA expression levels of Myogenin and β-actin as an internal standard. Statistical analysis was performed in the same method as that of Example 1. The results are shown in Fig. 3.

As shown in Fig. 3, the mRNA expression level of myogenin was concentration-dependently and remarkably enhanced by cytidylic acid. These results indicate that cytidylic acid has an excellent Myotube cell differentiation promoting effect.

### (Example 4) Myogenin Gene Expression Promoting Effect (II)

A level of Myogenin mRNA expression, a marker gene for myotube cell differentiation, was evaluated by the following method:
Cell culture and treatment with the test substances, total RNA extraction and reverse transcription reaction were performed as in Example 2. Real-time PCR analysis was used to determine mRNA expression levels of Myogenin and β-actin as an internal standard. Statistical analysis was also performed in the same method as that of Example 1. The results are shown in Figs. 4 and 5.

As shown in Fig. 4, the mRNA expression level of Myogenin was remarkably enhanced by the treatment with cytidylic acid and uridylic acid. As shown in Fig. 5, the mRNA expression level was remarkably enhanced by the treatment with cytidylic acid. These results indicate that cytidylic acid, uridylic acid and cytidine have an excellent myotube cell differentiation promoting effect.

### (Example 5) Myh7 Gene Expression Promoting Effect (I)

A level of Myh7 mRNA expression encoding a slow-muscle type myosin protein was evaluated using the following method:
Mouse myoblast cell line C2C12 cells were suspended in growth medium, seeded in 24-well plates, and incubated in a 37°C, 5% COz incubator until the cell density reached 70-90%. The growth medium was removed, and exchanged to a differentiation induction medium containing 5 mM disodium cytidylate or containing no cytidylic acid. The cells were cultured for 4 days while exchanging the medium once every 2 days, and the medium was then removed, and the total RNA extraction and reverse transcription reaction were performed as in Example 1. Real-time PCR analysis was used to determine mRNA expression levels of Myh7 and β-actin as an internal standard. Welch's t-test was used for statistical analysis. The threshold for statistical significance was set to 5%. The results are shown in Fig. 6.

As a result, the mRNA expression level of Myh7 was markedly enhanced by cytidylic acid, as shown in Fig. 6. These results indicate that cytidylic acid has an excellent promoting effect on slow-muscle type myosin protein expression.

### (Example 6) Myh7 Gene Expression Promoting Effect (II)

A level of Myh7 mRNA expression encoding a slow-muscle type myosin protein was evaluated using the following method:
Cell culture and treatment with the test substances, total RNA extraction and reverse transcription reaction were performed as in Example 2. Real-time PCR analysis was used to determine mRNA expression levels of Myh7 and β-actin as an internal standard. Statistical analysis was also performed in the same method as that of Example 1. The results are shown in Figs. 7 and 8.

As shown in Fig. 7, the mRNA expression level of Myh7 was remarkably enhanced by the treatment with cytidylic acid and uridylic acid. As shown in Fig. 8, the mRNA expression level of Myh7 was markedly enhanced by the treatment with cytidine. These results indicate that cytidylic acid, uridylic acid and cytidine have an excellent promoting effect on the slow-muscle type myosin protein expression.

### (Example 7) Mitochondrial DNA Copy Number Increasing Effect (I)

Cell culture and addition of test substance were carried out by the same method as that of Example 1. The cells were cultured for 6 days while exchanging a differentiation induction medium containing the test substance once every 2 days, the medium was then removed and total DNA was extracted from the cells using DNeasy Blood & Tissue kit (QIAGEN).

Extracted DNA, GoTaq(R) qPCR Master Mix (Promega) and a real-time PCR device, Thermal Cycler Dice Real Time System (Takara Bio), were used to determine COX2 (Cytochrome c oxidase subunit 2), a gene encoded in the mitochondrial DNA, and PPIA (Cyclophilin A), a gene encoded in human DNA as an internal standard.

The amount of COX2 present relative to the amount of PPIA present was evaluated as the mitochondrial DNA copy number. Statistical analysis was performed by the same method as that of Example 1. This Example was studied with n=3. The results are shown in Fig. 9.

As shown in Fig. 9, the mitochondrial DNA copy number was concentration-dependently and remarkably enhanced by cytidylic acid. These results indicate that cytidylic acid has an excellent mitochondria activating effect.

### (Example 8) Mitochondrial DNA Copy Number Increasing Effect (II)

Cell culture and addition of test substances were carried out in the same method as that of Example 2. The cells were cultured for 6 days, the medium was then removed and total DNA was extracted from the cells using DNeasy Blood & Tissue kit (QIAGEN).

Extracted DNA, GoTaq(R) qPCR Master Mix (Promega) and a real-time PCR device, Thermal Cycler Dice Real Time System (Takara Bio), were used to determine COX2 (Cytochrome c oxidase subunit 2), a gene encoded in the mitochondrial DNA, and PPIA (Cyclophilin A), a gene encoded in human DNA, as an internal standard.

The amount of COX2 present relative to the amount of PPIA present was evaluated as the mitochondrial DNA copy number. Statistical analysis was performed by the same method as that of Example 1. This Example was studied with n=3. The results are shown in Fig. 10.

As shown in Fig. 10, the mitochondrial DNA copy number was concentration-dependently and remarkably enhanced by the treatment with cytidylic acid and uridylic acid. These results indicate that cytidylic acid and uridylic acid have an excellent mitochondria activating effect.

### (Example 9) Myotube Cell Diameter Increasing Effect (I)

Cell culture and addition of a test substance were carried out by the same method as that of Example 1. The cells were cultured for 6 days while exchanging a differentiation inducing medium containing the test substance once every 2 days, and five positions near the center of the wells were then photographed at magnifications of 100. Using ImageJ image analysis software, the diameters of ten myotube cells per a photograph were measured in order from the myotube cell having the largest diameter, and an average of 50 cells was determined to be the diameter of the myotube cell in each well. Statistical analysis was performed by the same method as that of Example 1. Photographs of representative examples of the respective groups are shown in Fig. 11, and the measured results of the average myotube cell diameter are shown in Table 1 and Fig. 12.

**[Table 1]**

| | Control | 1mM CMP | 5mM CMP |
|---|---|---|---|
| Average (µm) | 53.9 | 61.1 | 62.5 |

As shown in Figs. 11 and 12, the myotube cell diameter was concentration-dependently and remarkably increased by cytidylic acid. These results indicate that cytidylic acid has an excellent myotube cell diameter increasing effect.

### (Example 10) Myotube Cell Diameter Increasing Effect (II)

Cell culture and addition of test substances were carried out by the same method as that of Example 2. The cells were cultured for 5-6 days while exchanging a differentiation inducing medium containing the test substances once every 2 days, and five positions near the center of the wells were then photographed at magnifications of 100. Photographs of representative examples of the respective groups are shown in Fig. 13.

Also, using ImageJ image analysis software, the diameters of ten myotube cells per a photograph were measured in order from the myotube cell having the largest diameter, and an average of 50 cells was determined to be the diameter of the myotube cell in each well. Statistical analysis was also performed by the same method as that of Example 2. These results are shown in Figs. 14 to 17.

As shown in Fig. 13, the myotube cell diameter was significantly increased by the treatment with cytidylic acid, uridylic acid, cytidine, and uridine.

As shown in Fig. 14, the myotube cell diameter was concentration-dependently and remarkably increased by the treatment with cytidylic acid. As shown in Fig. 15, the myotube cell diameter was significantly increased by the treatment with uridylic acid. As shown in Fig. 16, the myotube cell diameter was concentration-dependently and remarkably increased by the treatment with cytidine. As shown in Fig. 17, the myotube cell diameter was concentration-dependently and remarkably increased by the treatment with uridine. These results indicate that cytidylic acid, uridylic acid, cytidine, and uridine have an excellent myotube cell diameter increasing effect.

## Claims

1. A PGC-1α expression promoting agent comprising at least one pyrimidine nucleotide or a precursor thereof as active ingredient.

2. The PGC-1α expression promoting agent according to claim 1, wherein the pyrimidine nucleotide or the precursor thereof is one or more selected from the group consisting of cytidylic acid, uridylic acid, cytidine, and uridine.

3. A muscle-building agent comprising at least one pyrimidine nucleotide or a precursor thereof as an active ingredient.

4. The muscle-building agent according to claim 3, wherein the pyrimidine nucleotide or the precursor thereof is one or more selected from the group consisting of cytidylic acid, uridylic acid, cytidine, and uridine.

5. A mitochondria activating agent comprising at least one pyrimidine nucleotide or a precursor thereof as an active ingredient.

6. The mitochondria activating agent according to claim 5, wherein the pyrimidine nucleotide or the precursor thereof is one or more selected from the group consisting of cytidylic acid, uridylic acid, cytidine, and uridine.
